Europäisches Patentamt

(19) European Patent Office

Office européen des brevets

(11) Publication number: **0 296 036**
**A2**

## (12) EUROPEAN PATENT APPLICATION

(21) Application number: **88401425.9**

(22) Date of filing: **10.06.88**

(51) Int. Cl.4: **G 01 N 33/543**
**G 01 N 33/58**

(30) Priority: **15.06.87 IL 82873**

(43) Date of publication of application:
**21.12.88 Bulletin 88/51**

(84) Designated Contracting States:
**BE DE ES FR GB IT NL**

(71) Applicant: **Orgenics Ltd.**
**P.O. Box 360**
**Yavne 70650 (IL)**

(72) Inventor: **Fish, Falk**
**5 Kashani Street**
**Tel Aviv (IL)**

(74) Representative: **Orès, Bernard et al**
**Cabinet ORES 6, Avenue de Messine**
**F-75008 Paris (FR)**

(54) Reversed competitive solid phase immunoassay for detecting single epitope, analytes and kit therefor.

(57) The present invention relates to a solid phase competitive immunoassay method for detecting analytes, comprising :

(a) coating a surface with antibodies against the analyte to be determined;

(b) contacting the coated surface with an aqueous sample containing the analyte to be analyzed, and a conjugate of the analyte with a carrier so as to effect binding between

(i) the antibodies and the analyte, and

(ii) the antibodies and the analyte-carrier conjugate;

(c) removing the solution containing antibody-analyte and antibody-conjugate complexes; and

(d) measuring the amount of analyte-carrier conjugate remaining in the solution of step (c) to indicate the amount of the analyte in the sample.

EP 0 296 036 A2

## Description

## REVERSED COMPETITIVE SOLID PHASE IMMUNOASSAY FOR DETECTING SINGLE EPITOPE ANALYTES AND KIT THEREFOR

The present invention relates to a novel method of assaying biological materials to ascertain the content of specific substances, as well as to a kit for conducting such assays. More particularly, the invention relates to a method for detecting and measuring the presence of single epitope analytes in a biological sample.

Many conventional techniques for ascertaining the content of a particular drug, e.g., digoxin in urine, blood or the like, are known. One can effect complex chemical separations by chromatography and/or chemical reactions to separate the active material and then, determine the amount by weighing, by radioimmunoassay, or by other techniques. Immunoassay may be employed to detect and quantitatively measure the amounts of diverse analytes ranging from large (polymeric) molecules, such as proteins, polysaccharides, peptides, nucleic acids, and combinations thereof, to small organic molecules, such as drugs, hormones, antibiotics, toxicants, dyes, and the like. Generally, large molecules possess more than one binding site or epitope for an antibody. On the other hand, small molecules possess a single epitope, thus allowing the binding of only one antibody molecule to an analyte at any given time.

Most of the immunoassay techniques are heterogeneous, i.e., they require physical separation of the antibody-bound analyte from the non-bound analyte before further analysis can be done. A popular form of immunoassay, which is suitable for a large number of polymeric analyte molecules is of the "double sandwich" type, such as one described by ENGVALL and PERLMANN (Immunochemistry 9: 871-874, 1971). In this type of assay, the analyte-containing sample is contacted with an insolubilized antibody to the analyte. This usually takes the form of an antibody-covered test tube or a flat piece of plastic such as described in U.S. Patent Applications 619,739, 675,439, and 664,413.

The analyte binds to the insolubilized antibody through some of its epitopes. The unoccupied epitopes can then bind another antibody molecule, which is in the solution and is labelled by a biologically compatible label to generate an easily detectable signal. The labelling materials include radioactive isotopes, enzymes, fluorescent materials and combinations thereof. In this type of assay technique, the strength of the signal generated is proportional to the amount of analyte present in the sample.

However, single -epitope analytes cannot be measured by such an assay, because only one antibody molecule can bind to one molecule of the analyte. Therefore, as far as the heterogeneous assays for detecting single-epitope analytes are concerned, they have to be competitive.

The competitive immunoassay, based on the competition of an analyte with a constant quantity of labelled analyte on limited antibody receptors has been practiced widely since the 1960's (see Ekins, Br. Med. Bull. 30: 3-11, 1974). Catt and Tregear (Science 158: 1570-1572, 1967) demonstrated that antibodies irreversibly bind to polystyrene surfaces and therefore, could facilitate easy and quick separation between antibody-bound and free analytes. This was the foundation of all solid phase immunoassay, including the current ELISA.

Glad and Grubb (Anal. Biochem. 85: 180-187, 1978; 116: 330-340, 1981) elaborated on the principle of the competitive solid phase immunoassay in binding the antibody to the surfaces of capillaries and demonstrated that the distance of migration of a labelled analyte through such a capillary is directly proportional to the concentration of the competing analyte. Therefore, Glad and Grubb succeeded in obtaining a proportional signal (the distance of migration) in a competitive solid phase immunoassay. The principle of "Immunocapillary Migration" was utilized by Zuk et al. (Clin. Chem. 31: 1144-1150, 1985) in their Enzyme Immunochromatography assay in which a mixture of the tested analyte and a constant amount of analyte-enzyme conjugate migrates on a filter paper, to which antibodies specific to the analyte have been previously covalently bound. The distance of analyte-enzyme conjugate migration on the paper is proportional to the amount of analyte in the sample and is determined by immersing the paper in a chromogenic substrate cocktail.

U.S. Patents 3,646,346 (CATT) and 3,654,090 (SCHUURS et al.) disclose competitive immunoassay techniques for measuring the concentration of antigens in biological samples. British Patent 1,364,922 (GROSS) discloses an antigen for radioactive or fluorescent immunoassay. European Patent 135,071 (HENNING) discloses a chemiluminescent substituent for labelling a hapten to be assayed.

In a competitive immunoassay, a sample containing the analyte to be measured is contacted with an insolubilized antibody, as in the "double sandwich" assay. However, the signal is generated by the labelled analyte molecules, which are also a part of the system. The labelled analyte competes with the unlabelled analyte in the sample for the limited number of epitopes (antibody binding sites). Thus, if a large amount of the unlabelled analyte is present in the sample, a small amount of the labelled analyte will be bound to be insolubilized antibody, resulting in an inversely proportional signal, i.e., greater amount of the analyte in the sample will generate a smaller signal and vice-versa.

Although this technique may be satisfactory when the results are being analyzed by a professional with the aid of sophisticated computing devices, it is confusing to a layperson or a person with less skill and experience in the immunoassay field, or when attempts are made to interpret the results without complicated computations. Furthermore, the above-described techniques are inherently limiting in sensitivity, because (1) they lack an amplification system for the signal generated, and (2) if a lower amount of the analyte is to be measured, the fewer antibody receptors and fewer labelled competitor analyte should be provided, which reduces the intensity of the signal generated.

A homogeneous assay for detecting small analytes was introduced by Rubenstein and co-workers (Biochem. Biophys. Res. Comm. 47:846-851, 1972). In this assay, the analyte is contacted with soluble antibody and a soluble analyte-enzyme conjugate. When no analyte is present in the sample, all the antibodies in the system are free to bind to the analyte on the enzyme, resulting in an inhibition of the activity of the enzyme, which is measured by its ability to catalyze a chromogenic reaction. If the sample contains free analyte, it will neutralize the anti-analyte antibody in the system and will prevent its binding to the analyte-enzyme conjugate, resulting in heightened activity of the latter. In such an assay (EMIT = Enzyme Multiplied Immunoassay Technique), the signal (color) is proportional to the amount of the analyte.

A similar immunoassay technique is disclosed in U.S. Patent 4,477,576, DEUTSCH et al., whereby an enzymatic reaction of a bound conjugate is used to determine the concentration of free antigen remaining in the liquid phase. However, in the DEUTSCH process, the antibody is conjugated to an enzyme and originates in the liquid phase, and the antigen carrier conjugate is immobilized on a solid matrix.

U.S. Patent 4,298,685, PARIKH et al., utilizes a biotin-avidin systems, with one of these, specifically avidin, is immobilized. However, in this technique the other member of the set, biotin, is conjugated to antigen. Furthermore, measurement is ultimately taken by means of the conventional enzyme-labelled antigen. However, because these assays are conducted in a fluid phase and involve variations in the rate of activity of an enzyme, color measuring devices and some computations are required to analyze the results.

Accordingly, there is a need for an immunoassay technique for detecting and measuring single-epitope analytes in a biological sample, which generates an amplified signal and is easy to operate by individuals of low skill and sophistication.

The present invention attempts to overcome the above-mentioned disadvantages by offering a solid phase assay for single-epitope analytes generating a signal, which is proportional in magnitude to the amount of an analyte in a sample. When this invention is utilized in an ImmunoComb environment (U.S. patent applications 619,739, 675,439 and 664,413), a multi-analyte, instrument-free assay kit for small analytes may be constructed.

The invention utilizes the ability of solid phase competitive assays to detect single-epitope analytes with the amplification of signals offered by double-sandwich solid phase assay technique for detecting multi-epitope analytes.

According to the present invention, an analyte containing sample is contacted with an insolubilized antibody and a conjugate of the analyte with a carrier. The analyte in the sample competes with the analyte-carrier conjugate for the limited insoluble antibody binding sites. Therefore, the greater the amount of analyte is present in the sample, the greater amount of analyte-carrier conjugate will stay in the solution, i.e., unbound. The amount of the conjugate remaining in the solution is then assessed by either (a) transferring the solution to another reaction vessel and assaying for the amount of the carrier, or (b) by inserting an insoluble device carrying receptors specific for the conjugate into the solution containing unbound conjugates.

The above-mentioned advantages of the instant reversed competitive solid phase immunoassay for detecting single-epitope analytes will be more clearly understood by the detailed description of the invention provided hereinbelow in conjunction with the accompanying drawings, in which:

Figure 1 is a schematic illustration of the system in a state when no analyte is present in the sample;

Figure 2 is a schematic illustration of the system in a state when the analyte is present in the sample;

Figure 3 is a schematic illustration of the system in a state when an insoluble device carrying the receptors specific for the conjugate, is inserted into the reaction solution;

Figure 4 is a schematic illustration of the system in a state when the complexes of (a) the antibody and the analyte, and (b) the antibody and the analyte-carrier conjugate are formed in the solution;

Figures 5 illustrates the system according to another embodiment of the invention in which multiple single-epitope analyte are measured.

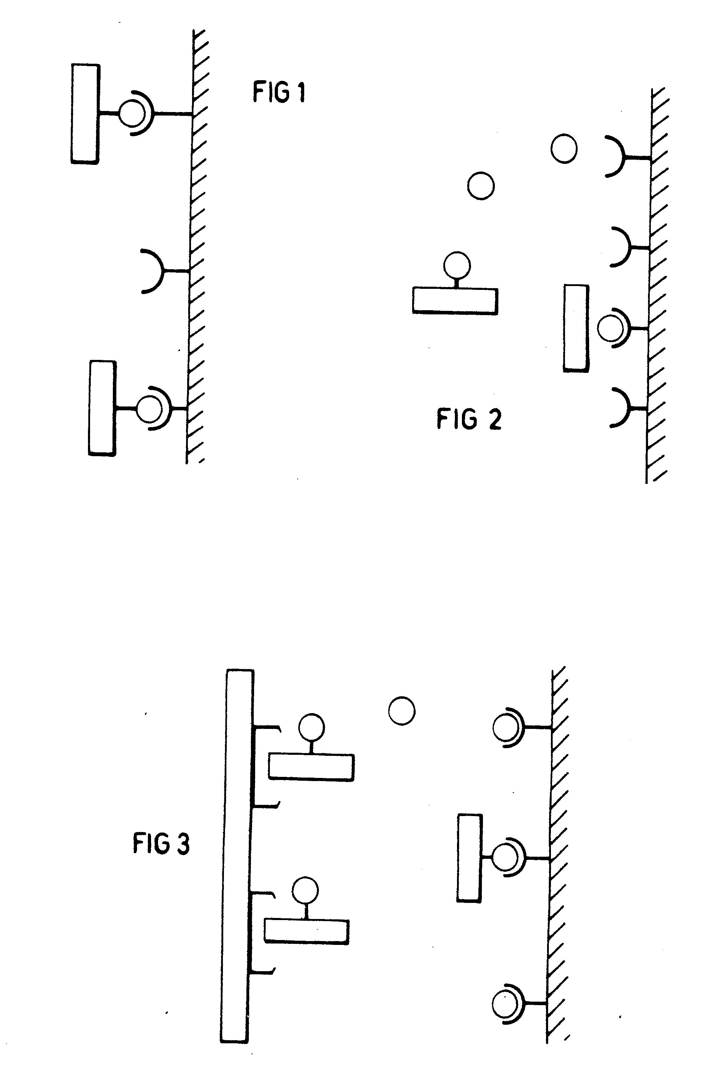

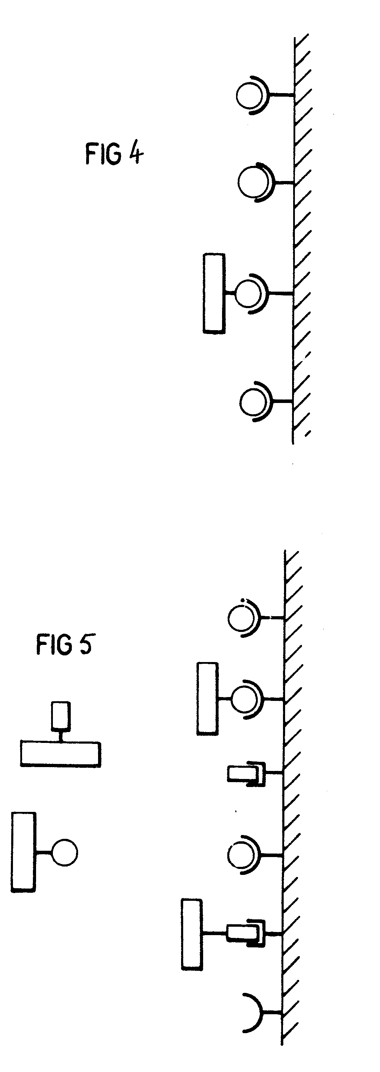

In accordance with the invention, an immunoassay method and a kit therefor are provided for determining single-epitope analyte(s) in a biological sample suspected of containing the analyte(s), whereby the sample is contacted with an insolubilized antibody specific for the analyte and a conjugate of the analyte with a carrier.

In carrying out the preparation sequence of the present invention, the following procedure can be employed. The solid support or interior surface of a reaction vessel or test tube of the type described in pending Israeli patent applications Nos. 75464 and 77144 (incorporated herein in their entirety by reference thereto), capable of absorbing antibodies against the analyte to be measured, is contacted with a dilute solution of the antibodies such that a sufficient amount of antibodies is adsorbed on the solid surface. Antibodies may be prepared by injecting conjugates of the analyte with a protein molecule such as serum albumin or egg albumin. An example for preparing a conjugate of propranolol with bovine serum albumin is detailed by Wang et al., FEBS Letters, vol. 199, Number 2, pages 173-178, 1986. Other methods for preparing conjugates of small organic molecules with larger biological polymers can be found in Methods in Enzymology, vol. 70, pages 85-104 and 151,159, Academic Press, New York, 1980.

Following injection series, antibodies against the analyte in question are generated. The exact schedule of injections and ancillary materials to enhance the immune response are well-known to anyone sufficiently experienced in the art of raising antibodies. If the immunized animals are mice or rats, monoclonal antibodies may be obtained, as described in the example of Wang et al. mentioned above. From larger animals blood is drawn and antibodies are isolated from the serum by various protocols which are familiar to all immunologists.

The antibodies are usually suspended to a final concentration of 0.01 to 10000 ug/ml, preferably 5-200 ug/ml in a solution containing various solutes such as 0.1M bicarbonate, pH 9.0-10.0, or: phosphate buffered saline, pH 7.0-7.5, or: unbuffered saline or any other solute combination which enhances binding of antibody to a solid support. The binding of the antibody to the surface of the tube may be spontaneous (i.e. the solution of the antibody is dispensed into the tube and incubated there for periods of time ranging from 1 second to 48 hours, preferably 1-24 hours. Proteins and antibodies bind to the surface and do not come off upon washing in aqueous solutions or dilute detergents. Following binding of the antibodies to the surface, the surface is treated to prevent any further binding of proteins. Such treatments are also well-known in the art and include solutions of albumin, diluted sera, milk, detergents and combinations thereof. Antibodies may also be chemically coupled to the surface. Some protocols are detailed by P. Tijssen in his monograph "Practice and Theory of Enzyme Immunoassays" published by Elsevier Science Publishers, Amsterdam, Holland.

In another test tube, the biological sample containing the analyte(s) to be measured is prepared. The sample can be a biological fluid, originating from a living organism (urine, blood, serum, plasma, cerebrospinal fluid, tears, saliva), environment obtained sample (water, water following passage of air), and plant origin samples. The low molecular weight analytes that may be detected and measured can belong to the groups of drugs, hormones, carcinogens (e.g. aflatoxins, polychlorinated biphenols), lipids and related materials, sphingolipids, nucleotides, nucleosides, organic bases, antiobiotics, porphyrines, steroides, alkaloids and vitamins. The list is, of course, not limiting and is provided as an example only.

In a third test tube, a known quantity of a conjugate of the analyte to be measured and a carrier for the conjugate are prepared. The conjugates are prepared by various methods well-known in the art. Some examples are detailed in Methods in Enzymology, vol. 70, pages 85-104 and 151-159, Academic Press, New York, 1980, in P. Tijssen in his monograph "Practice and Theory of Enzyme Immunoassays" published by Elsevier Science Publishers, Amsterdam, Holland; in Wang et al., FEBS Letters, vol. 199, Number 2, pages 173-178, 1986. The carrier molecule may be a mono-, oligo- or polymer of peptides, saccharides, nucleotides or any combinations thereof, or any other suitable materials such as vitamins, dyes, etc. For example, dextran, ficoll®, starch, alginic acid, carrageenan, various gums, albumins of various origins, globulins of various origins, gelatin, polyamino acids, random copolymers of amino acids, DNA, RNA, Aluminum hydroxide or oxide, titanium hydroxide or chloride, polyvinyl pyrrolidone and other polymers. The list is not limiting. Although it is not necessary, however, it is preferable that the carrier molecule be antigenic, i.e., has the ability to bind antibodies. In case the carrier is not antigenic, it is possible to conjugate to it another chemical compound as for example DNP, phenol trymitrophenyl flurescein, biotine and others which can serve as an antigen. The assay of the unbound conjugate in the last phase of the assay will be executed by assaying for the conjugated analyte only.

During measurement, the biological sample and the analyte-carrier conjugate, respectively, are mixed to prepare an aqueous sample. The aqueous sample is then slowly poured in the first tube having antibodies adsorbed on the interior surface thereof. The mixture thus obtained, is allowed to stand for periods of 1 minute to 24 hours, preferably 1-60 minutes at temperatures between 0-40°C, preferably 5-25°C.

The analyte(s) in the sample competes with the analyte-carrier conjugate for the limited amount of antibodies present on the solid surface, and immunlogical binding between (i) the antibodies and the analyte, and (ii) the antibodies and the analyte-carrier conjugate occurs. Due to the immunological competition between the analyte and the conjugate, if the sample contains a greater amount of the analyte then, an increased binding between the antibodies and the analyte occurs and thus, a greater amount of the conjugate stays in the solution in the unbound state. The solution containing the antibody-analyte and the antibody-conjugate is then transferred to another reaction vessel for measuring the amount of the analyte-carrier conjugate remaining in the solution.

The amount of the unbound conjugate is measured by assaying the solution for the amount of carrier and for analyte by a regular noncompetitive solid phase enzyme immunoassay as described in P. Tijssen in his monograph "Practice and Theory of Enzyme Immunoassays" published by Elsevier Science Publishers, Amsterdam, Holland, or by inserting into the solution of unbound conjugate an insoluble device which carries receptors for the conjugate. The insoluble device may be the kind of device disclosed in pending Israel patent application Nos. 75464 and 77144 (incorporated herein in their entirety by reference thereto). The receptor may, for example, be an antibody specific to the carrier component of the conjugate or a specific ligand to the carrier, e.g., biotin in the case of avidin or a lectin in the case of specific sugars, an anti-immunoglobulin antibody in case the carrier is an immunoglobulin, an anti-albumin antibody in case the carrier is an albumin.

In another embodiment of the invention, the instant method may be employed for determining multiple single epitope analytes in a given biological sample. The sample containing the analytes is contacted with a solid phase coated with a mixture of antibodies specific to the analytes and a series of analyte-carrier conjugates, in which each analyte is conjugated to a distinct carrier. For example, the first analyte will be conjugated to bovine serum albumin, a second analyte to egg albumin, and a third analyte to Keyhole Limpet hemocyanine. There is no immunological cross reactivity among those three potential carriers, so they can all be assayed at the same time. All of the displaced analyte-carrier conjugates are then measured by a multi-analyte card of the type described in pending Israeli patent application No. 75464 and 77144 (incorporated herein in their entirety by reference thereto).

The instant invention is advantageous and improves upon the principle of solid phase competitive immunoassays and their derivatives as follows:

1. There is no requirement for the covalent binding of antibody to a specialized surface. The classical adsorption of antibody to plastic can be utilized. Non-purified and even unseparated whole immune serum can be utilized.

2. The interfacing of the competitive immunoassay with the ImmunoComb principle allows for amplification of the signal, for example, by introducing a labelled antibody to the carrier. Therefore, the system may be adapted to obtain higher sensitivity by simply adding "layers" of antibodies.

3. In the present invention analytes need not be conjugated to an enzyme, with the requirement that the conjugation reaction results in inactivation of the enzyme. Therefore, any molecule, small or large, may employed as carrier, as long as a receptor to the carrier can be devised. Since the receptor need not be an antibody, other ligand-receptor systems, e.g., biotin-avidin, sugar-lectin may be employed.

4. A multi-analyte assay can be devised by adsorbing a mixture of antibodies onto the reaction vessel's surface (these antibodies can be produced even in a single animal) and by conjugating each of the analytes to a different carrier. The ImmunoComb device then will carry specific receptors for each of the carriers. A single enzyme label can then be utilized for final visualization.

It will be understood that the specification and examples are illustrative, but not limitative of the present invention and that other embodiments within the scope and spirit of the invention will be apparent to those skilled in the art.

**Claims**

1. A solid phase competitive immunoassay method for detecting analytes, comprising:
    (a) coating a surface with antibodies against the analyte to be determined;
    (b) contacting the coated surface with an aqueous sample containing the analyte to be analyzed, and a conjugate of the analyte with a carrier so as to effect binding between
    (i) the antibodies and the analyte, and
    (ii) the antibodies and the analyte-carrier conjugate;
    (c) removing the solution containing antibody-analyte and antibody-conjugate complexes; and
    (d) measuring the amount of analyte-carrier conjugate remaining in the solution of step (c) to indicate the amount of the analyte in the sample.

2. The assay method of claim 1, wherein said analyte to be analyzed is a single-epitope analyte.

3. The assay method of claim 1, wherein said carrier is selected from the group consisting of a peptide, a saccharide, a nucleotide, and a combination thereof.

4. The assay method of claim 3, wherein said peptide is a monomer.

5. The assay method of claim 3, wherein said peptide is a oligomer.

6. The assay method of claim 3, wherein said peptide is a polymer.

7. The assay method of claim 1, wherein said carrier is an antigen.

8. The assay method of claim 3, wherein said saccharide is a monosaccharide.

9. The assay method of claim 3, wherein said saccharide is a disaccharide.

10. The assay method of claim 3, wherein said saccharide is a polysaccharide.

11. The assay method of claim 3, wherein said nucleotide is a polynucleotide.

12. The assay method of claim 1, wherein said carrier is a vitamin.

13. The assay method of claim 1, wherein said carrier is a dye.

14. A solid phase competitive immunoassay method for detecting single-epitope analytes, comprising:
    (a) coating by adsorption at least a part of the internal surface of a test tube with antibodies against the analyte to be determined;
    (b) contacting the coated surface of the test tube with an aqueous sample containing the analyte to be analyzed, and a conjugate of the analyte with a carrier so as to effect binding between
    (i) the antibodies and the analyte, and
    (ii) the antibodies and the analyte-carrier conjugate;
    (c) inserting into the test tube an insoluble device carrying thereon receptors specific for the analyte-carrier conjugate so as to effect binding between the unbound conjugate and the receptor; and
    (d) measuring the amount of the analyte-carrier conjugate bound to the receptor to indicate the amount of the analyte in the sample.

15. The assay method of claim 14, wherein said carrier is selected from the group consisting of a peptide, a saccharide, a nucleotide, and a combination thereof.

16. The assay method of claim 15, wherein said peptide is a monomer.

17. The assay method of claim 15, wherein said peptide is a oligomer.

18. The assay method of claim 15, wherein said peptide is a polymer.

19. The assay method of claim 14, wherein said carrier is an antigen.

20. The assay method of claim 15, wherein said saccharide is a monosaccharide.

21. The assay method of claim 15, wherein said saccharide is a disaccharide.

22. The assay method of claim 15, wherein said saccharide is a polysaccharide.

23. The assay method of claim 15, wherein said nucleotide is a polynucleotide.

24. The assay method of claim 14, wherein said carrier is a vitamin.

25. The assay method of claim 14, wherein said carrier is a dye.

26. A solid phase competitive immunoassay method for detecting multiple single-epitope analytes, comprising:

(a) coating least a part of a solid phase with a mixture of antibodies against various types of analytes to be determined;

(b) preparing an aqueous sample containing the analytes to be determined and a mixture of various conjugates of the analytes with a carrier for each of the conjugates;

(c) contacting the solid phase with the sample of (b) so as to effect binding between (i) various types of antibodies and their corresponding analytes, and (ii) various types of antibodies and their corresponding analyte-carrier conjugates; and

(d) measuring an amount of each type of the analyte-carrier conjugate remaining in the solution by a pre-established method to indicate the amount of various types of analytes in the sample.

27. The assay method of claim 26, wherein said carrier is selected from the group consisting of a peptide, a saccharide, a nucleotide, and a combination thereof.

28. The assay method of claim 27, wherein said peptide is a monomer.

29. The assay method of claim 27, wherein said peptide is a oligomer.

30. The assay method of claim 27, wherein said peptide is a polymer.

31. The assay method of claim 26, wherein said carrier is an antigen.

32. The assay method of claim 27, wherein said saccharide is a monosaccharide.

33. The assay method of claim 27 wherein said saccharide is a disaccharide.

34. The assay method of claim 27, wherein said saccharide is a polysaccharide.

35. The assay method of claim 27, wherein said nucleotide is a polynucleotide.

36. The assay method of claim 26, wherein said carrier is a vitamin.

37. The assay method of claim 26, wherein said carrier is a dye.

38. A kit for a solid phase competitive immunoassay method for detecting a single-epitope analyte in a biological sample, comprising:

(a) a first reaction vessel for containing a known quantity of said biological sample;

(b) a second reaction vessel for containing a known quantity of antibodies against said analyte to be quantitated;

(c) a third reaction vessel containing a known quantity of conjugate of said analyte and a carrier for said conjugate, said carrier being selected from the group consisting of a peptide, a saccharide, a nucleotide, and a combination thereof; and

(d) an insoluble device for carrying thereon a known quantity of receptors specific for the analyte-carrier conjugate, wherein said quantity of antibodies is sufficient to bind a substantial quantity of said analyte and said conjugate, said quantity of receptors is sufficient to bind a substantial quantity of said analyte-carrier conjugate.

39. The assay kit of claim 38, wherein said receptor is an antibody specific to said conjugate.

40. The assay kit of claim 38, wherein said receptor is a ligand specific to said carrier.

FIG 1

FIG 2

FIG 3

0296036

FIG 4

FIG 5